# EUROPEAN PATENT APPLICATION

(11) **EP 1 380 315 A1**
(43) Date of publication of application: **14.01.2004**
(21) Application number: 03012190.9
(22) Date of filing: 04.06.2003
(51) Int. Cl.: A61M 5/32, A61B 5/15

(54) **Safety needle assembly**

(30) Priority: 12.06.2002 US 170318; 14.06.2002 US 173239
(71) Applicant: Becton, Dickinson and Company, Franklin Lakes, New Jersey 07417-1880 (US)
(72) Inventor: MacLean Crawford, Jamieson William, New York, NY 10025 (US); Bennett, Michael, Summit, New Jersey 07901 (US); Newby, C. Mark, Tuxedo, NY 10987 (US)
(74) Representative: Müller-Boré & Partner Patentanwälte

(57) **Abstract**

The present invention is a safety shield assembly having a shield and a collar for connecting the shield to a fluid handling device whereby the shield may be pivoted with respect to the collar. Preferably, the safety shield assembly may be used with a needle assembly, an intravenous infusion set a syringe, a catheter or other fluid handling devices or assemblies that contain piercing elements. The shield includes a clip with a plurality of cannula finger locks for preventing re-exposure of the used needle.

## Description

### FIELD OF THE INVENTION

The present invention relates to a shield for a needle and more particularly to a safety shield assembly that may be used in conjunction with a syringe assembly, a hypodermic needle, a needle assembly, a needle assembly with a needle holder, a blood collection needle, a blood collection set, an intravenous infusion set or other fluid handing devices or assemblies that contain piercing elements.

### BACKGROUND OF THE INVENTION

Disposable medical devices having piercing elements for administering a medication or withdrawing a fluid, such as hypodermic needles, blood collecting needles, fluid handling needles and assemblies thereof, require safe and convenient handling. The piercing elements include, for example, pointed needle cannula or blunt ended cannula.

Safe and convenient handling of disposable medical devices is recognized by those in the medical arts so as to minimize exposure to blood borne pathogens. Safe and convenient handling of disposable medical devices results in the disposal of the medical devices intact.

As a result of this recognition, numerous devices have been developed for shielding needles after use. Many of these devices are somewhat complex and costly. In addition, many of these devices are cumbersome to use in performing procedures. Furthermore, some of the devices are so specific that they preclude use of the device in certain procedures or with certain devices and/or assemblies. For example, some devices employ very short thin needle cannulas. A shield designed to lock near the distal end of one needle cannula might not engage a much shorter needle cannula. Additionally, a shield designed to lock with a wider gauge needle cannula might be more likely to generate a spray upon engaging a much narrower needle cannula. Furthermore, it may be desirable to reduce the force required to effect shielding without reducing the audible and tactile indications of complete shielding.

Therefore, there exists a need for a safety shield assembly: (i) that is manufactured easily; (ii) that is applicable to many devices; (iii) that is simple to use with one hand; (iv) that can be disposed of safely; (v) that does not interfere with normal practices of needle use; (vi) that has tactile features whereby the user may be deterred from contacting the needle, the user may easily orient the needle with the patient and easily actuate and engage the shield assembly; (vii) that has visual features whereby the user may be deterred from contacting the needle, the user may easily orient the needle with the patient and easily actuate and engage the shield assembly; (viii) that is not bulky; (ix) that includes means for minimizing exposure to the user of residual fluid leaking from the needle; and (x) provides minimal exposure to the user because the needle shield is immediately initiated by the user after the needle is withdrawn from the patient's vein.

### SUMMARY OF THE INVENTION

The present invention is a safety shield assembly that comprises: a shield; means for connecting the shield to a fluid handling device that contains a piercing element, such as needle; means for pivoting the shield away from the needle and means for securely covering and/or containing the needle within the shield.

Preferably, the shield comprises a rearward end, a forward end, a slot or longitudinal opening for housing the used needle in the forward end, means for securing the needle in the slot, means for guiding the needle into the slot, means for connecting the shield and the fluid handling device, means for guiding the user's fingers to move the shield into various positions, and means for retaining the shield securely over the used needle.

Desirably, the means for connecting the shield to the fluid handling device is a collar. Preferably, the shield is connected movably to a collar which is connected to a fluid handling device.

Preferably, the shield is connected to the collar by a hanger bar that engages with a hook arm on the collar so that the shield may be pivoted with respect to the collar into several positions. It is within the purview of the present invention to include any structure for connecting the shield to the collar so that the shield may be pivoted with respect to the collar. These structures include known mechanical hinges and various linkages, living hinges, or combinations of hinges and linkages.

Most preferably, the shield is connected to the collar by an interference fit between the hanger bar and the hook bar. Therefore, the shield always is oriented in a stable position and will not move forward or backwards unless movement of the shield relative to the hanger bar and the hook bar is initiated by the user.

Alternatively, the shield and collar may be a unitary one-piece structure. The one-piece structure may be obtained by many methods, including molding the shield and the collar as a one-piece unit, thereby eliminating the separate shield and collar during the manufacturing assembly process.

The assembly of the present invention may further comprise tactile and visual means for deterring the user from contacting the needle, providing easy orientation of the needle with the patient and providing the user with a guide for actuation and engagement with the shield.

The assembly of the present invention may further comprise means for minimizing exposure by the user to residual fluid leaking from a used needle. For example, a polymer material, such as a gel, may be located in the shield.

Most desirably, the assembly of the present invention is such that the cooperating parts of the assembly provide the means for the shield to move into a forward position over the needle. Thus, by simple movement of the shield into a forward position over the used needle, the assembly is ready for subsequent disposal. Therefore, the safety shield assembly of the present invention provides minimal exposure of the user to a needle because the shielding is initiated by the user immediately after the needle is withdrawn from the patient's vein.

Desirably, the assembly of the present invention may be used with a syringe assembly, a hypodermic needle, a needle assembly, a needle assembly with a needle holder, a blood collection set, an intravenous infusion set or other fluid handling devices. Preferably, the assembly of the present invention is used with a needle assembly comprising a needle and a hub. Preferably the needle is a conventional double ended needle.

Most preferably, the present invention is used with a needle assembly comprising a hub and a needle connected to the hub whereby the needle comprises a non-patient end and an intravenous end. The collar of the present invention may comprise a hook arm and the shield may be connected movably to the hook arm. Thus the shield may be pivoted with respect to the collar and moved easily into several positions.

Preferably, the collar is fitted non-rotatably with the hub of the needle assembly. Additionally, the collar includes cooperating means that mate with reciprocal means on the shield to provide a clear audible and tactile indication of complete shielding. The cooperating means on the collar may include generally chevron-shaped projection formed on a side of the collar substantially diametrically opposite the hook arm or other such structure that provides the hinge connection to the shield. The chevron-shaped structure includes a forward or distal point. Slanting surfaces diverge and extend proximally from the distal point. The slanting surfaces cooperate with the reciprocal means on the shield to generate a deflection of the sidewalls of the shield away from one another. The chevron-shaped structure further includes proximal ends that are convexly arcuate. The convexly arcuate ends of the chevron-shaped structure on the collar cooperate with the reciprocal means on the shield and with the resiliently deflectable sidewalls of the shield to generate the tactile and audible indication of shielding.

The means for securely locking the shield in the second position comprises a clip secured to the interior of the shield. The clip preferably includes an elongate base with means for attachment to the shield and a plurality cannula finger locks extending from the base for locked engagement with the cannula when the shield is in the second position around the needle cannula. Each cannula finger lock preferably includes a support leg that projects from the base and a cannula engaging leg or detent that extends from the free end of the support leg. Each cannula finger lock is dimensioned, disposed and aligned to contact the needle cannula when the shield approaches the second position. Contact between the cannula and each cannula finger lock may cause each cannula finger lock and/or the needle cannula to deflect resiliently. Sufficient rotation of the shield will cause the needle cannula to pass the cannula engaging legs. As a result, each cannula finger lock and/or the needle cannula will resiliently return to or toward its undeflected condition for securely trapping the needle cannula in the shield. The means for attaching the clip to the shield may comprise apertures in the shield and snaps or other locking projections formed on the base of the clip. The snaps can be force fit through the apertures in the shield. Alternatively, the means for the clip to the shield may comprise apertures in the clip and snaps or other locking projections formed on the shield. The number and location of the cannula finger locks may be selected in view of the length of the needle. At least two and preferably three cannula finger locks may be provided.

In an alternate embodiment, the shield preferably includes a proximal or rearward portion that is hingedly connected to the collar and a distal or forward portion that comprises a support extending unitarily from the rearward portion of the shield. The shield may further comprise a cannula channel securely mounted to at least the support of the forward portion. The channel may comprise a top wall securely connected to the support of the shield and a pair of opposed spaced apart sidewalls extending downwardly from the top wall. The spacing between the sidewalls is selected to accommodate the needle cannula in the channel. At least one of the sidewalls of the cannula channel is formed with at least one cannula finger lock that projects angularly toward the top wall of the cannula channel. The cannula finger lock is dimensioned, disposed and aligned to contact the needle cannula when the shield approaches the second position. Contact between the needle cannula and the cannula finger lock will cause the cannula finger lock to resiliently deflect toward the sidewall from which the cannula finger lock extends. Sufficient rotation of the shield will cause the needle cannula to pass the cannula finger lock. As a result, the cannula finger lock will resiliently return to or toward its undeflected condition for securely trapping the needle cannula in the shield.

The top wall of the cannula channel may comprise means for engaging reciprocal means on the support of the forward portion of the shield. The mounting means on the cannula channel may be disposed in a non-central position relative to the longitudinal direction of the channel. Thus, the cannula channel can be mounted to the shield support in either of two alternate orientations that are rotated 180° from one another. In a first rotational orientation, the cannula channel projects distally or forwardly beyond the support of the shield. In a second rotational orientation, the cannula channel projects rearwadly or proximally from the support of the shield to overlie the rearward portion of the shield. Thus, the first rotational orientation of the cannula channel ensures that the pointed distal end of a long needle cannula is surrounded by the cannula channel and engaged by the cannula finger locks. The second rotational orientation of the cannula channel ensures that a shorter needle cannula is surrounded by the cannula channel and engaged by the cannula finger locks.

The cannula channel may have a proximal or rear end configured for engaging the collar or hub when the shield is in its second position. Engagement of the channel with the collar or hub may contribute to locked retention of the shield in the second position. Additionally, engagement of the proximal end of the cannula channel with the collar or hub may further support the channel and prevent excessive deformation of the channel.

Preferably, the collar is fitted with the hub of the needle assembly so that the collar cannot rotate around the hub. Alternatively, the collar and hub may be a unitary one-piece structure. The one piece structure may be accomplished by many methods including molding the collar and the hub as a one-piece unit thereby eliminating the need to separately assemble the collar to the hub during the manufacturing process.

Most preferably, the collar is fitted with the hub of the needle assembly so that the bevel surface or bevel up surface of the intravenous or distal end of the needle faces the same side of the collar when the shield is in the first position. Alignment of the collar, hub, shield and needle with the bevel surface up makes it easier to insert the needle into the patient without manipulating the assembly. The orientation of the intravenous end of the needle with the bevel up assures the user that the needle is properly oriented for use and does not require any manipulation before use. Most notably, the orientation of the shield provides a visual indication to the user of the orientation of the bevel surface of the needle.

Preferably, the shield is capable of pivoting from a first position where the intravenous end of the needle is exposed and bevel up, to an intermediate position where the needle is partially covered, to a second position where the needle is covered completely.

Alternatively, it is within the purview of the present invention that the shield, collar and hub is a unitary one-piece structure. The one-piece structure may be accomplished by many methods including molding the shield, collar and hub as a one-piece unit thereby eliminating the need to separately assemble the shield, collar and hub during the manufacturing process.

It is an advantage of the present invention that the shield covering the used intravenous end of the needle provides easy containment of the used needle. A further advantage of the shield is that it will only move upon initiation by the user.

The assembly of the present invention when used with a fluid handling device is also easily disposable when removed from a conventional needle holder, or other such device.

A notable attribute of the present invention is that it is easily adaptable with many devices. For example, the invention is usable with syringe assemblies, hypodermic needles, needle holders, blood collection needles, blood collection sets, intravenous infusion sets such as catheters or other fluid handling devices or assemblies that contain piercing elements.

Another notable attribute of the present invention is that the tactile and visual features deter the user from touching the needle, allow the user to easily orient the needle with the patient and guide the user to actuate and engage the shield of the assembly.

### BRIEF DESCRIPTION OF THE DRAWINGS

FIG. 1 is a perspective view of the safety shield assembly of the present invention as connected to a needle assembly and related packaging features.

FIG. 2 is a perspective view of the unassembled pieces of FIG. 1.

FIG. 3 is a bottom view ofthe shield as shown in FIG. 2.

FIG. 4 is a cross sectional view of the collar as shown in of FIG. 2 taken along lines 4-4 thereof.

FIG. 5 is a cross sectional view of the needle hub as shown in FIG. 2 taken along lines 5-5 thereof.

FIG. 6 is a cross sectional view of the shield of FIG. 2 taken along lines 6-6 thereof.

FIGS. 7-12 illustrate the use of the safety shield assembly with the needle assembly of FIG. 1 with a conventional needle holder.

FIG. 13 is a cross sectional view of the assemblies in use with a conventional needle holder as shown in FIG. 12 taken along lines 13-13 thereof.

FIG. 14 is a cross-sectional view of the assemblies of FIG. 13 taken along lines 14-14 thereof.

FIG. 15 is a bottom view of the assemblies as shown in FIG. 11.

FIG. 16 illustrates an additional embodiment of the present invention, whereby a gel material is located in the shield as shown in a bottom view of the assemblies of FIG. 11.

FIG. 17 is a perspective view of an additional embodiment of the present invention in use with a blood collection set.

FIG. 18 is a perspective view of an additional embodiment of the present invention in use with a syringe.

FIG. 19 is a perspective view of an additional embodiment of the present invention in use with a catheter.

FIG. 20 is a bottom view similar to FIG. 15, but showing an additional embodiment of the present invention.

FIG. 21 is a perspective view of an alternate safety shield assembly as connected to a needle assembly and related packaging features.

FIG. 22 is a perspective view of the unassembled pieces of FIG. 21.

FIG. 23A is a bottom view of the shield as shown in FIG. 22.

FIG. 23B is an enlarged bottom view of an ear of the shield.

FIG. 24A is a cross sectional view of the shield of FIG. 22 taken along lines 24A-24A thereof.

FIGS. 24B is a cross-sectional view similar to FIG. 24A, but showing the cannula channel in an end-to-end reversed orientation.

FIG. 25 is a longitudinal cross-sectional view of the assemblies in use with a conventional needle holder.

FIG. 26 is a cross-sectional view of the assemblies of FIG. 25 taken along lines 26-26 thereof.

FIG. 27 is an exploded side elevational view of an additional embodiment of the present invention.

FIG. 28 is a bottom view of the clip of FIG. 27.

### DETAILED DESCRIPTION OF THE INVENTION

While this invention is satisfied by embodiments in many different forms, there is shown in the drawings and will herein be described in detail, the preferred embodiments of the invention, with the understanding that the present disclosure is to be considered as exemplary of the principles of the invention and is not intended to limit the invention to the embodiments illustrated. Various other modifications will be apparent to and readily made by those skilled in the art without departing from the scope and spirit of the invention. The scope of the invention will be measured by the appended claims and their equivalents.

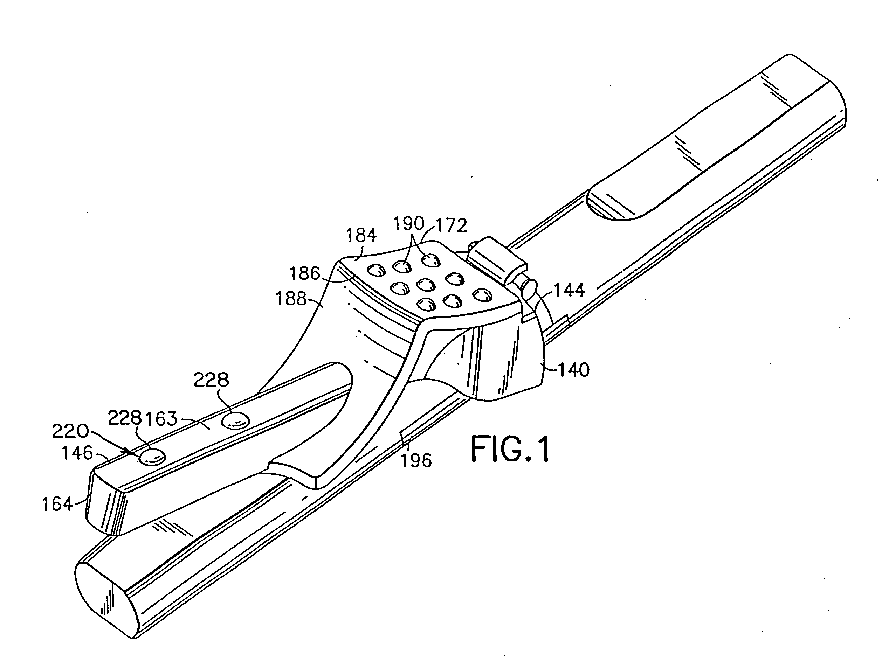

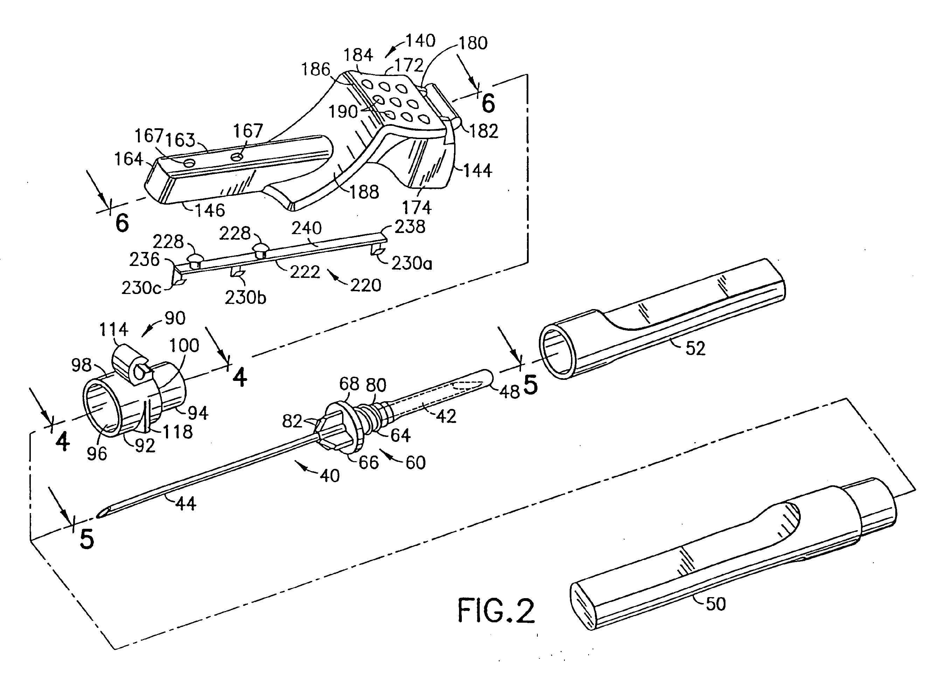

Referring to the drawings in which like reference characters refer to like parts throughout the several views thereof, FIGS. 1 and 2 illustrate a needle assembly with the safety shield assembly of the present invention and the related packaging features. The needle assembly includes a needle **40**, a hub **60**, packaging features to cover the needle and a label. The safety shield assembly includes a collar **90** and a shield **140**.

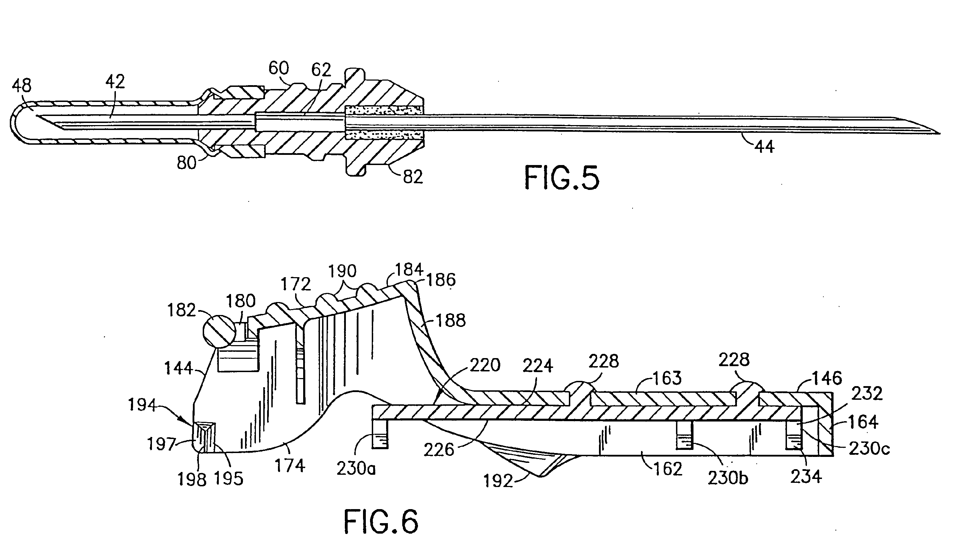

As shown in FIG. 2 and 5, needle **40** includes a non-patient end **42**, an intravenous end **44** and a passageway **46** extending between the non-patient end and the intravenous end. An elastomeric sleeve **48** covers the non-patient end. A first rigid sleeve **50** covers the intravenous end and a second rigid sleeve **52** covers the non-patient end and the elastomeric sleeve. As shown in FIG. 1, a label **196** may also be applied to the finally assembled parts.

As shown in FIGS. 2 and 5, hub **60** includes a threaded end **64**, a ribbed end **66** and passageway **62** extending between the threaded end and the ribbed end. Threaded end **64** and ribbed end **66** are separated by flange **68**. Non-patient end **42** of needle **40** extends from threaded end **64** and intravenous end **44** of needle **40** extends from ribbed end **66**. Preferably, threaded end **64** comprises male threads **80** for mounting the hub on a conventional needle holder and ribbed end **66** comprises male ribs **82** for connecting the hub and collar **90**.

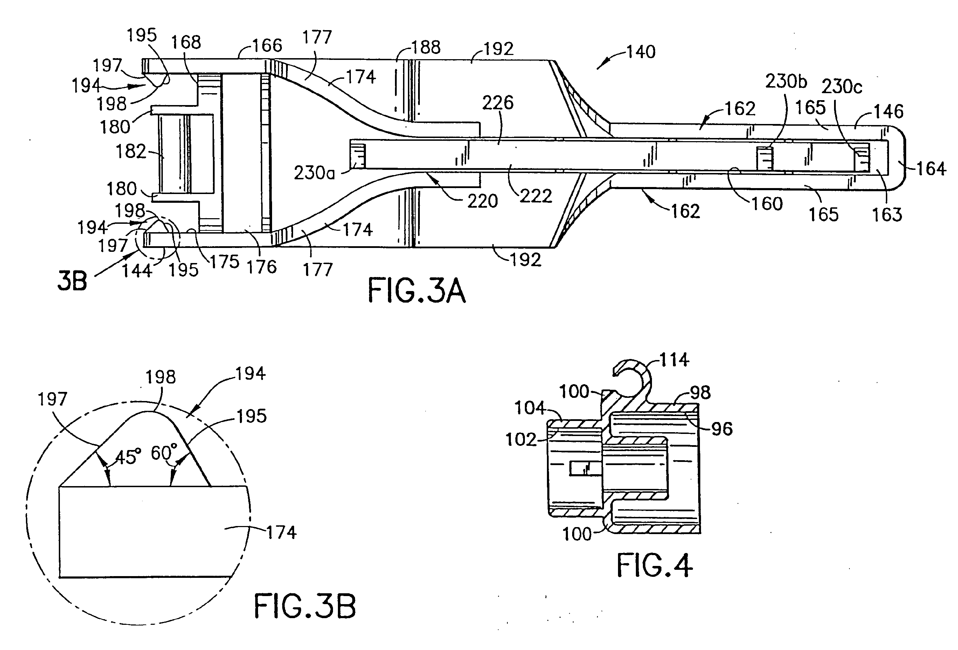

As shown in FIGS. 2 and 4, collar **90** includes a forward skirt **92** and a rearward skirt **94**. Forward skirt **92** is cylindrical and comprises an inner circumferential surface **96** and an outer circumferential surface **98**. Forward skirt **92** mates with rearward skirt **94** at a shoulder **100**. Rearward skirt **94** is cylindrical and comprises an inner-circumferential surface **102** and an outer circumferential surface **104** and extends from shoulder **100** opposite of forward skirt **92**. The inner diameter of forward skirt **92** is larger than the inner diameter of rearward skirt **94**. Alternatively, the inner diameters for collar **90** can be equal. A hook **114** extends from outer circumferential surface **98** of forward skirt **92**. Additionally a chevron-shaped protrusion **118** projects outwardly from outer circumferential surface **98** of forward skirt **92** at a side opposite hook **114**. The chevron-shape protrusion **118** is substantially symmetrical and has a peak **120** pointed toward forward skirt **92** and ramp surfaces **122** that diverge symmetrically from peak **120** toward rearward skirt **94**. Ramp surfaces **122** terminate at rounded ends **124** at the outer side and proximal extremes of chevron-shaped protrusion **118**. Rounded ends **124** extend continuously into the proximal side of chevron-shaped protrusion **118** facing toward rearward skirt **94**.

As shown in FIGS. 2 and 6, shield **140** comprises a rearward end **144** and a forward end **146**. Forward end **146** of shield **140** includes a slot or longitudinal opening **160** formed by sidewalls **162** that extend downwardly from top wall **163** and run substantially opposite of one another in parallel along the length of slot **160** towards forward end wall **164**. Slot **160** is slightly wider than needle **40**. Sidewalls **162** include bottom edges **165** that extend substantially parallel to one another and parallel to top wall **163**. Top wall **163** is formed to include two longitudinally spaced apertures **167** extending entirely therethrough.

Shield **140** further includes a clip **220**. Clip **220** includes an elongate base **222** having a width less than the distance between sidewalls **162** at forward end **146** of shield **140**. Base **222** includes opposite top and bottom surfaces **224** and **226**. Top surface **224** is configured to nest closely with the inner surface of top wall **163** of shield **140**. Top surface **224** is characterized by mounting projections **228**. Mounting projections **228** are disposed and configured to be snap fit through apertures **167** in top wall **163** of shield **140**. More particularly, each mounting projection **228** includes a head that is tapered to a cross-sectional dimension slightly greater than the inside cross-sectional dimension of the respective aperture **167** in top wall **163**. Thus, the head of mounting projection **228** and portions of top wall **163** adjacent apertures **167** will yield slightly as mounting projections **228** are forced through apertures **167**. Top wall **163** and mounting projections **228** will return resiliently to their initial position after the enlarged heads passed through apertures **167**. Hence, base **222** of clip **220** will be held securely adjacent top wall **163** of shield **140**.

Clip **220** is characterized further by a plurality of cannula finger locks **230a**, **230b** and **230c** projecting from bottom surface **226** of base **222**. Each cannula finger lock **230** includes a support leg **232** and a locking finger **234**. Cannula finger locks **230** can take any of several optional constructions. For example, support leg **232** can be substantially rigid with respect to base **222** and locking finger **234** can be substantially rigid with respect to support leg **232**. In these situations, cannula finger locks **230** will function to deflect needle **40** resiliently and then to trap needle **40** beneath locking finger **234**. Alternatively, the support leg **232** can be resiliently deflectable relative to base **222**. Thus, the support leg **232** may deflect relative to base in response to forces generated by contact with needle **40**. Support leg **232** then will return resiliently to an undeflected condition so that locking finger **234** engages and traps needle **40**. This embodiment requires base **220** to be sufficiently narrow to permit deflection of support leg **232**. As a further option, locking finger **234** may be configured to deflect resiliently relative to support leg **232**. Still further, both support leg **232** and locking finger **234** may be resiliently deflectable.

Base **222**, as shown most clearly in FIGS. 3 and 6 is longer than top wall **163** of forward end **146** of shield **140**. Hence, base **222** includes a distal end **236** near forward end wall **164** and a proximal end **238** that extends into rearward end **144** of shield **140**. Cannula finger lock **230a** is disposed near proximal end **238** of base **222** and in rearward end **144** of shield **140**. As a result, proximal cannula finger lock **230a** is particularly useful for needle assemblies that employ a very short needle **40**. Of course, however, cannula finger locks **230b** and **230c** also can be employed in situations where needle **40** is longer.

Broken line **240** in FIG. 2 shows an optional proximal end for base **222**. Base **222** with a proximal end at line **240** can be used with needle assemblies known to employ the long needle **40**. In this embodiment, base **222** does not extend into rearward end **144** of shield **140**.

Rearward end **144** of shield **140a** is substantially identical to rearward end **144** of shield **140**. Thus, a repetitive description is not provided and corresponding elements merely are identified by the same reference numerals.

The extreme rear ends of sidewalls **174** on collar engaging area **166** include rounded ears **194** that project toward one another from opposed inner surfaces **175** of sidewalls **174**. Rounded ears **194** are disposed to engage chevron-shaped projection **118** on collar **90**. More particularly, each rounded ear **194** includes a distal surface **195**, a proximal surface **197** and a curved surface **198** extending between distal and proximal surfaces **195** and **197**. Distal surface **194** is aligned to sidewall **174** at a rake angle of approximately 60° and proximal surface **197** is aligned to sidewall **174** at an angle of approximately 45°. Curved surface **198** extends smoothly and convexly between distal and proximal surfaces **195** and **197**. Proximal surfaces **197** of rounded ears **194** will engage ramp surfaces **122** of chevron-shaped projection **118** to deflect sidewalls **174** slightly away from one another as shield **140** approaches the second position. The apex of curved surface **198** on each rounded ear **194** passes the respective rounded end surface **124** on chevron-shaped projection **118** on collar **90**. As a result, sidewalls **174** begin to return resiliently toward an undeflected condition. The resilient return of sidewalls **174** and raked distal surface **195** of ears **194** also causes sidewalls **174** to snap against chevron-shaped projection **118**. This snapping action provides a clear audible and tactile indication of complete shielding and occurs substantially when the used needle is trapped by cannula finger locks **230**. The angles of distal and proximal surfaces **195** and **197** of rounded ears **194** affects the performance of shield **140**. In particular, a smaller acute angle alignment of proximal face **197** reduces the force required to move shield **140** passed rounded ears **194**. A larger acute angle proximal surface **197** of rounded ears **194** requires a greater force to move shield **140** toward the second position. Similarly, the angle between distal surface **195** and sidewall **174** affects the acceleration characteristics as shield **140** is propelled toward the second position in response to the resilient return of sidewalls **174** and hence affects the audible indication of shielding.

Top finger guide area **172** comprises a first ramp **184** that extends slightly on an upwardly slope from the rearward end of the collar engaging area to a shoulder **186**. From shoulder **186** extends a second ramp **188** which slopes downwardly towards top section **163**. Most preferably, first ramp **184** comprises touch bumps **190**. The touch bumps provide a tactile and visual guide to alert the user that the user's finger has contacted the shield and that the shield is in a defined or controlled position. The touch bumps may be any configuration so long as they extend and are distinct from the top finger guide area. The touch bumps may also be of a distinguishing color as compared to the top finger guide area or the shield.

Second ramp **188** has interior surface **192** for urging the needle toward the center of slot **160** as the shield is being rotated into the second position. The exterior surfaces are slightly inclined and extending radially from the second ramp. The interior surfaces are especially helpful if the longitudinal axis of the needle is misaligned with respect to the longitudinal axis of the hub.

Extending arms **180** are located at rearward end **168** and at the beginning of top finger area **172** and hold hanger bar **182**.

The safety shield assembly and the needle assembly are assembled together whereby needle **40** is connected to hub **60** and sealed with adhesive at the ends of the hub. Hub **60** is then joined with collar **90** by ultra-sonic welding techniques or any other bonding techniques, or mechanical fit, whereby rearward annular skirt **94** of collar **90** mates with ribbed end **66** of the hub. Male ribs **82** of the hub are contained or forced fitted within inner sidewall **102** of rearward annular skirt **94** of collar **90**. The collar is aligned with the intravenous end of the needle whereby the hook arm is aligned with the bevel up of the needle. Then rigid sleeve **50** is force fitted into inner side wall **96** of forward skirt **92** of collar **90** to cover the needle. Clip **220** is mounted into shield **140** by forcing mounting projections **228** through mounting apertures **167** in shield **140**. Thereafter, shield **140** is connected to collar **90** whereby hanger bar **182** is force fitted into hook member **114** whereby slot **160** faces rigid sleeve **50**. Most preferably, the shield is connected to the collar by a force fit or interference fit between the hanger bar and the hook bar. Therefore, the shield is always oriented in a stable position and will not move unless movement of the shield is positively initiated by the user. To assemble the last piece, shield **140** is moved towards rigid sleeve **50** and second rigid sleeve **52** is force fitted onto outer sidewall **104** of rearward skirt **94** of collar **90**.

In addition, a label **196** may be applied to the finally assembled parts. The label may be used to provide tamper resistance of the parts, so that they are not reused.

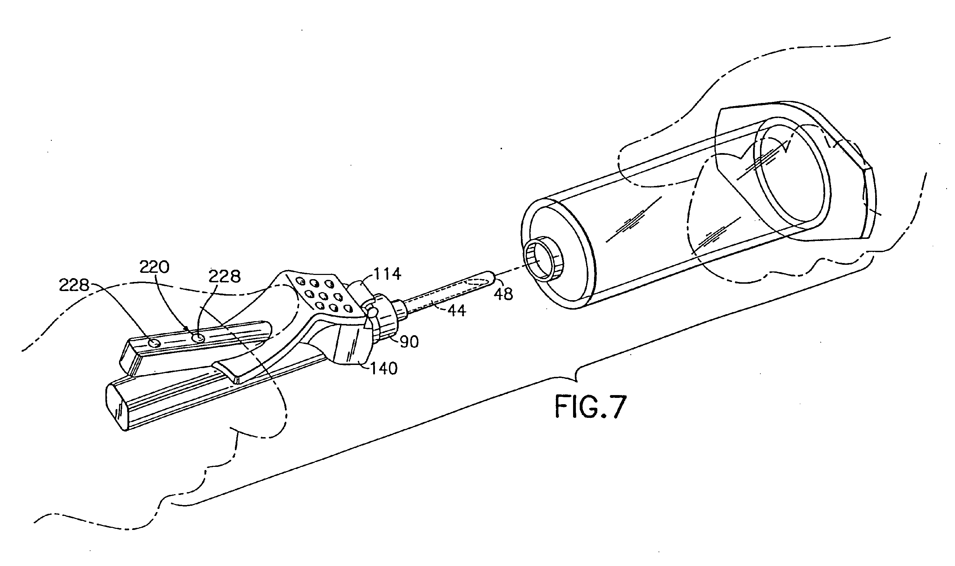

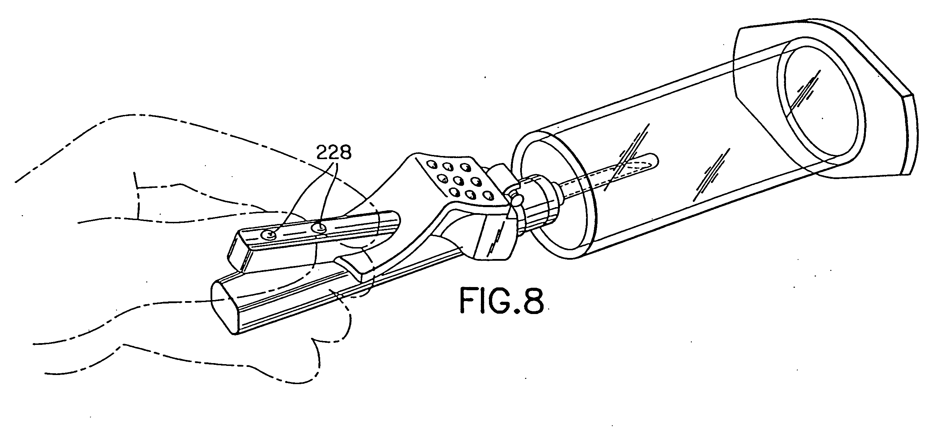

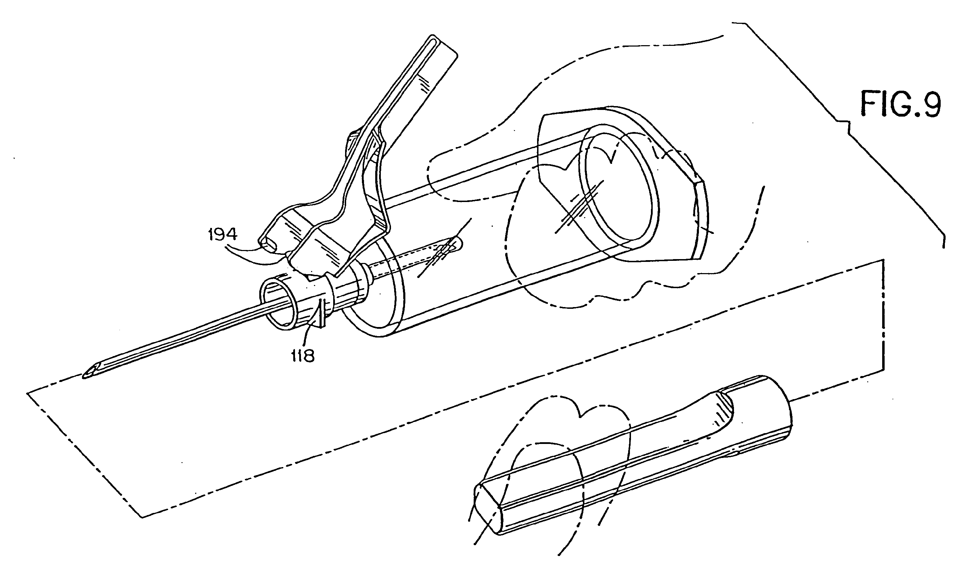

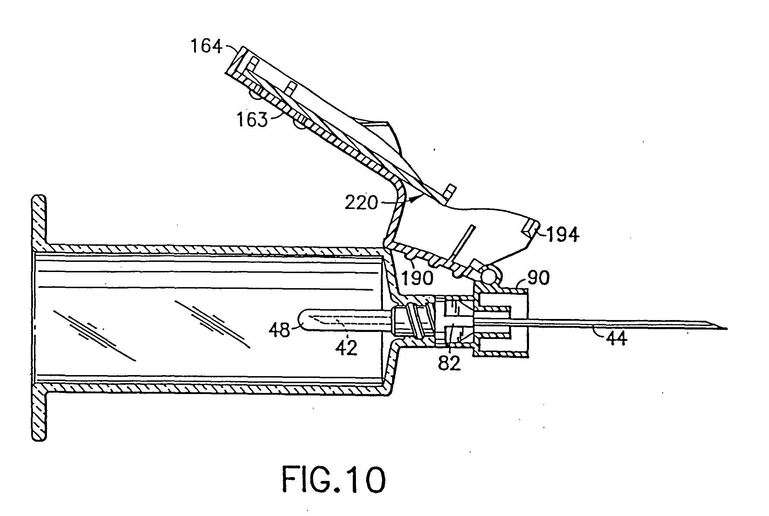

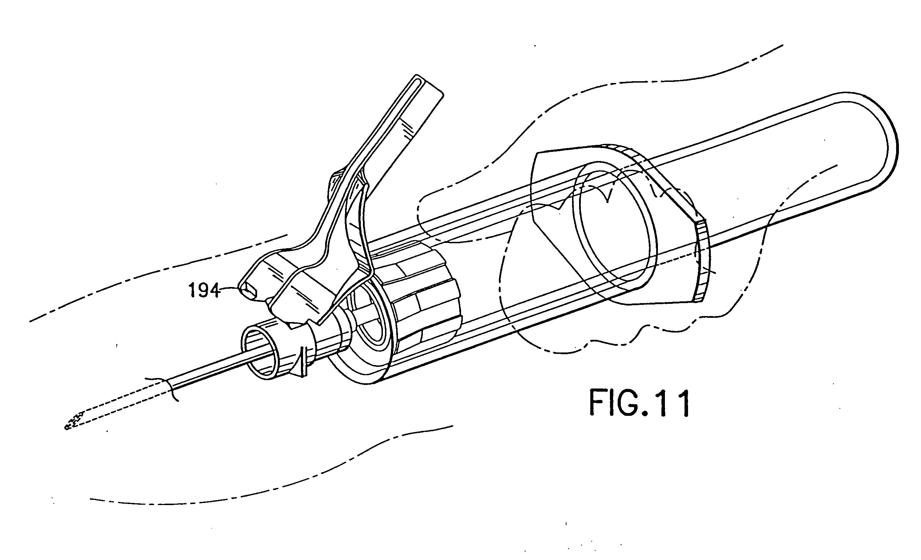

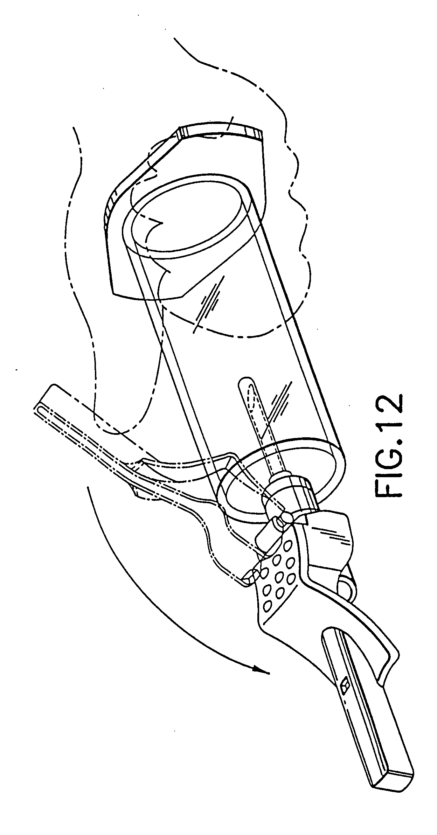

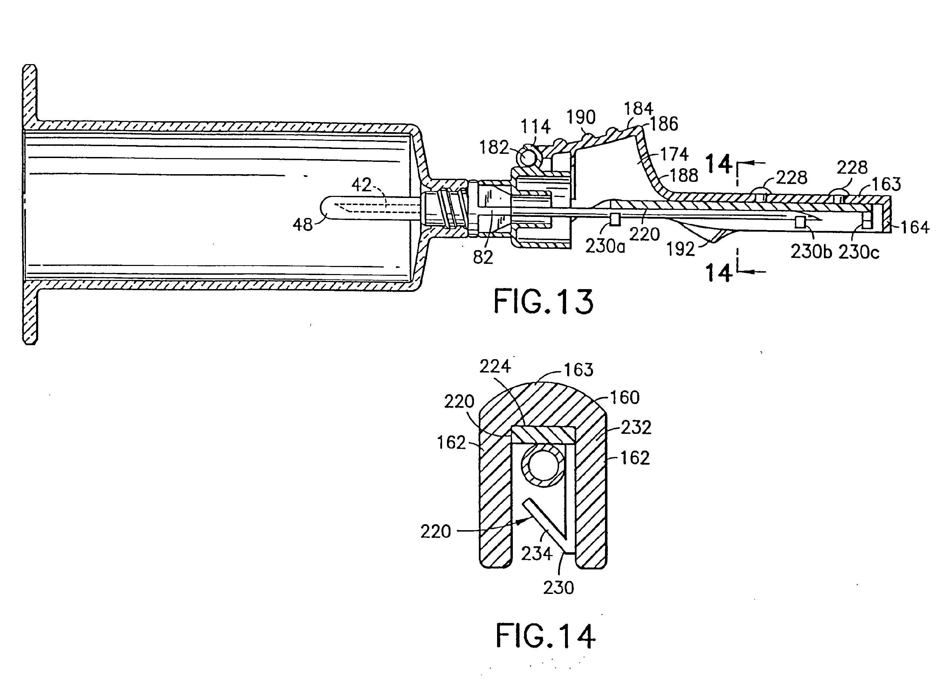

In use, as shown in FIGS. 7-15, the non-patient needle shield is removed and then a needle holder is screwed onto the hub of the needle. As specifically shown in FIGS. 8 and 12 the shield is then rotated back by the user towards the needle holder. Then as shown in FIG. 9, the intravenous needle shield is removed from covering the intravenous needle. Then as shown in FIG. 10, a venipuncture is conducted whereby the intravenous end of the needle is inserted into a vein of a patient and an evacuated tube having a closure is inserted into the needle holder. Then as shown in FIGS. 11 and 13, when the venipuncture is complete the user easily rotates the shield from the first position towards the intravenous needle to an intermediate position and then the user pushes on the shield at the top finger guide area to move the shield into a second position whereby the needle is trapped in the longitudinal opening. More particularly, needle **40** contacts cannula finger locks **230a-c** of clip **220**. The engagement of needle **40** with cannula finger locks **230a-c** causes cannula finger locks **230a-c** to deflect sufficiently to pass needle **40**. As a result, cannula finger locks **230a-c** will return resiliently to an undeflected condition. Thus, needle **40** will be trapped between cannula finger locks **230a-c** and base **222**. The deflection of cannula finger locks **230a-c** can occur in the support legs **232** and/or the locking fingers **234**. Alternatively, cannula finger locks **230a-c** can be substantially rigid structures that generate deflection of needle **40** sufficient for needle **40** to pass locking fingers **234**. Needle **40** then will return resilient to an undeflected condition and will be trapped between locking fingers **234** and base **222**.

Needle **40** is contained within shield **140** as the shield is pivoted into the second position. More particularly, proximal surfaces **197** of rounded ears **194** move over ramp surfaces **122** of chevron-shaped projection **118** and cause sidewalls **174** to deflect away from one another. The angularly aligned proximal faces **197** of rounded ears **194** ensure easy movement of shield **140**. Additionally, the resiliency of sidewalls **174** and the angular alignment of distal surface **195** of ears **194** causes a cooperation with rounded proximal ends **124** of chevron-shaped projection **118** to accelerate shield **140**. This accelerated movement of shield **140** helps to generate a clear audible and tactile indication of complete shielding.

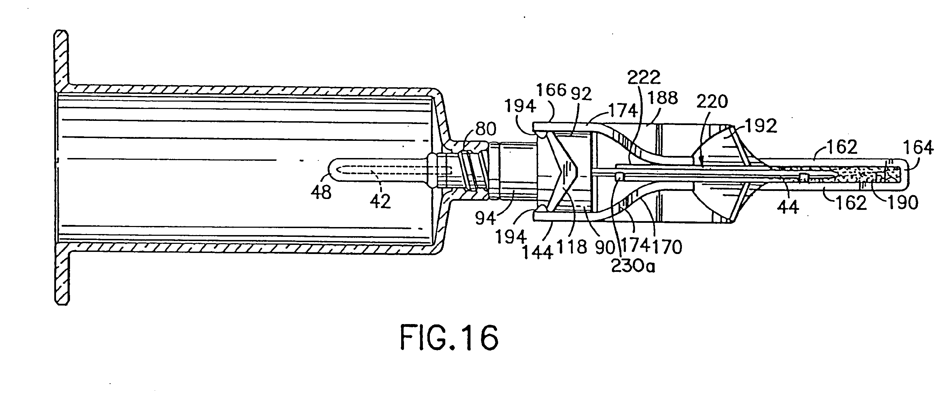

Alternatively as shown in FIG. 16, a gel material **190** is located in shield **140** so that when the needle snaps past cannula finger locks **230** it will come to rest in gel material **190**. The gel material will contain any residual fluid that may be on the needle. Simultaneously, rounded ears or projections **198** move over rounded proximal ends **124** of chevron-shaped projection **118**. This causes sidewalls **174** to deflect away from one another and then to snap back into engagement with collar **90** to provide a clear audible and tactile indication of complete shielding.

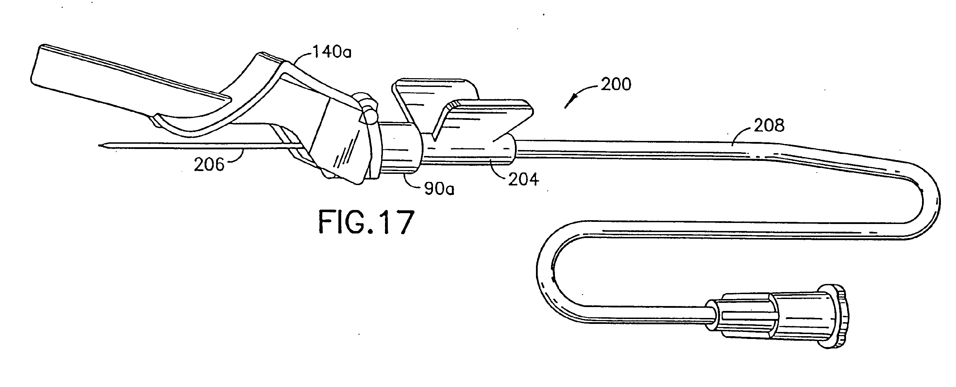

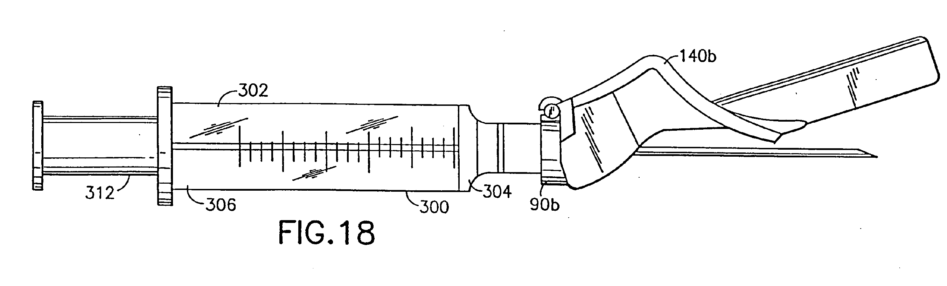

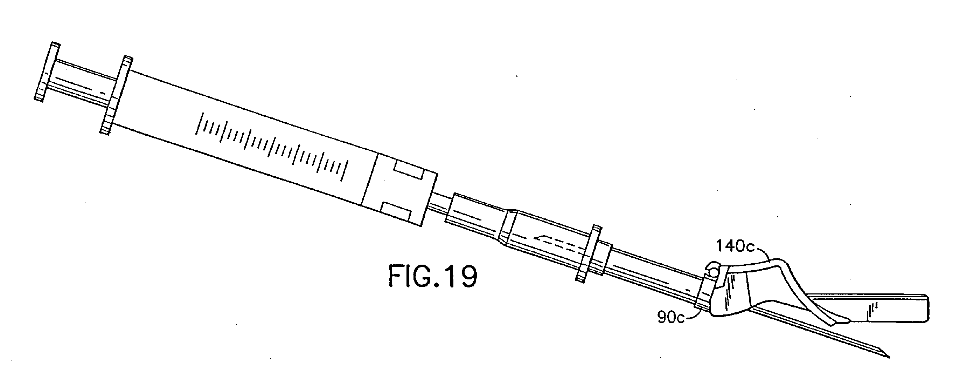

FIGS. 17, 18, and 19 are further embodiments of the invention that may include components which are substantially identical to the components of FIGS. 1-3. Accordingly, similar components performing similar functions will be numbered identically to those components of FIGS. 1-3, except that a suffix "a" will be used to identify those similar components in FIG. 17, a suffix "b" will be used to identify those similar components in FIG. 18 and a suffix "c" will be used to identify those similar components in FIG. 19.

Alternatively, the safety shield assembly of the present invention may be used in conjunction with a conventional intravenous (IV) infusion set, as illustrated in FIG. 17.

For purposes of illustration, shield **140a** and collar **90a** are connected to a conventional IV infusion set, **200**, or butterfly structure comprising a needle body with a needle hub **204** extending from the forward end of the needle body and a needle **206** embedded in hub **204**. Extending from the rearward end of the needle body is flexible tubing **208** which is conventional and utilized to allow the user to manipulate the structure and to connect it subsequently to supplies of infusion liquids or for the return of collected blood if the arrangement is being used to collect blood.

Infusion set **200** further comprises flexible wings **210** attached to and projecting outwardly from needle hub **204**.

Alternatively, the safety shield assembly of the present invention may be used in conjunction with a syringe, as illustrated in FIG. 18.

For purposes of illustration, shield **140b** and collar **90b** are connected to a conventional hypodermic syringe **300** comprising a syringe barrel **302** having a distal end **304** a proximal end **306** and a plunger **312**.

Alternatively, the present invention may be used in conjunction with a catheter as illustrated in FIG. 19.

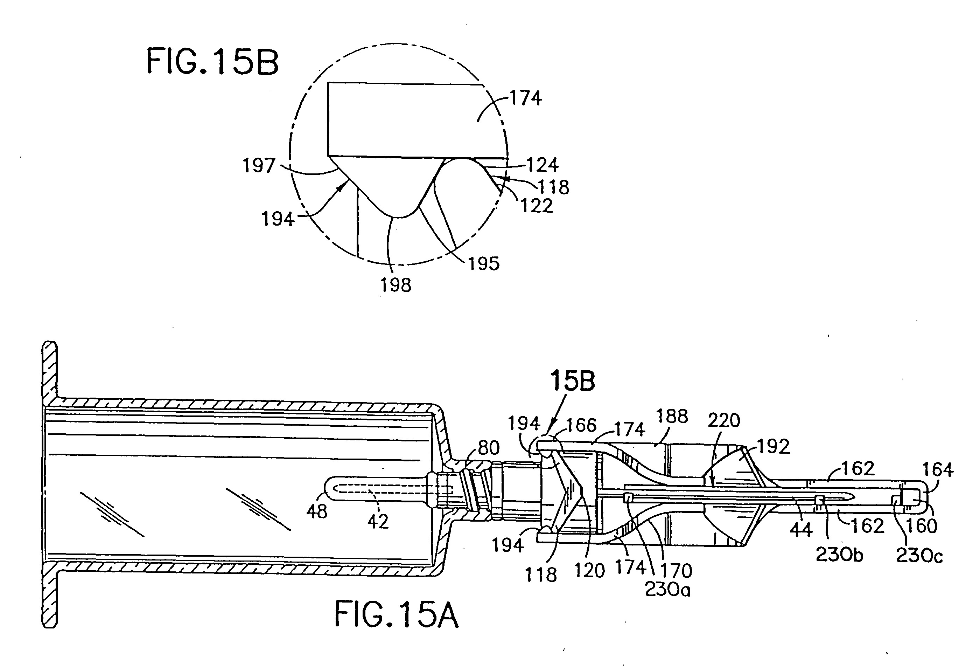

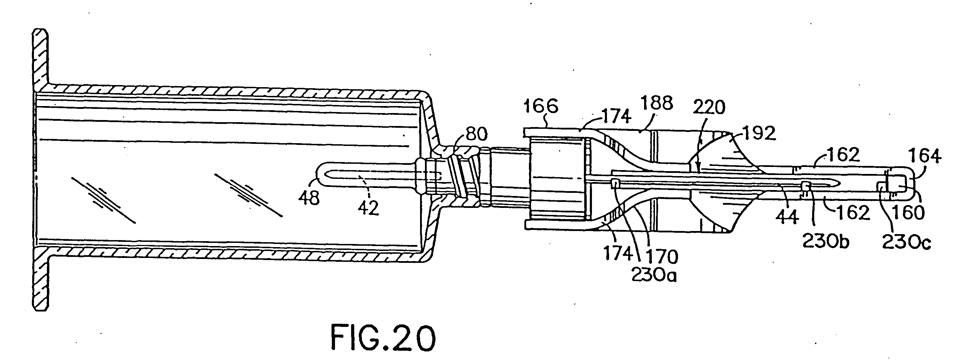

A further alternate embodiment is illustrated in FIG. 20, and is virtually identical to the embodiment of the invention depicted in FIG. 15A. As a result, comparable numerals have been employed to identify identical or very similar components. FIG. 20, however, differs from FIG. 15A in that collar **90** does not have the chevron-shaped protrusion **118** illustrated in FIG. 15A. Additionally, shield **140** does not have ears comparable to rounded ears **194** of FIG. 15A. Thus, the embodiment illustrated in FIG. 20 relies entirely upon the engagement of cannula finger locks **220** with needle **40**. There are fewer structures on the embodiment of FIG. 20 to achieve the audible and tactile indication of complete shielding, as in the previous embodiment, and no structure for accelerating shield **140** into the second position around needle **40**. However, upon complete shielding, the retention between shield **140** and needle **40** in the embodiment of FIG. 20 is comparable to the attention achieved by the previous embodiments.

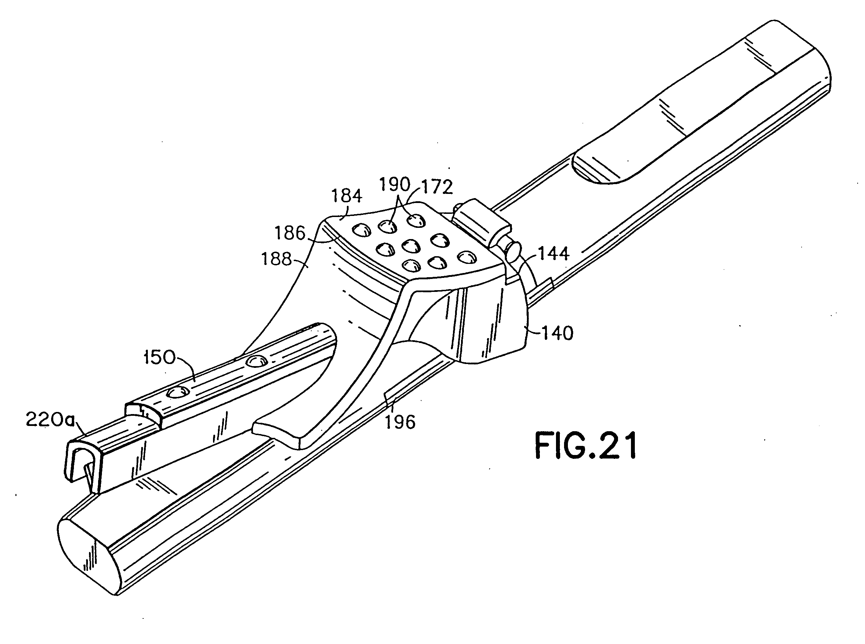

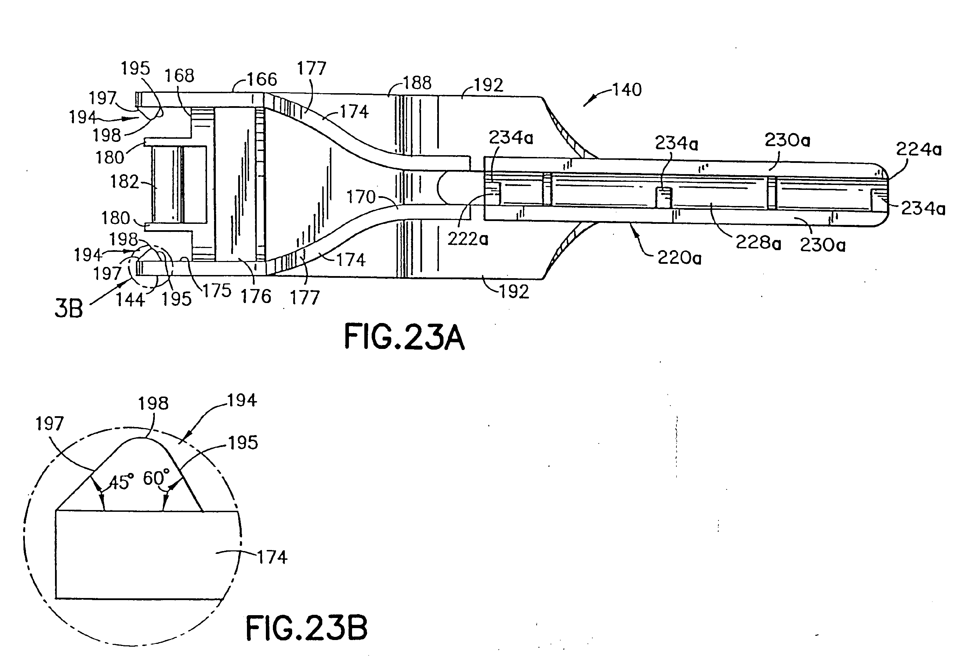

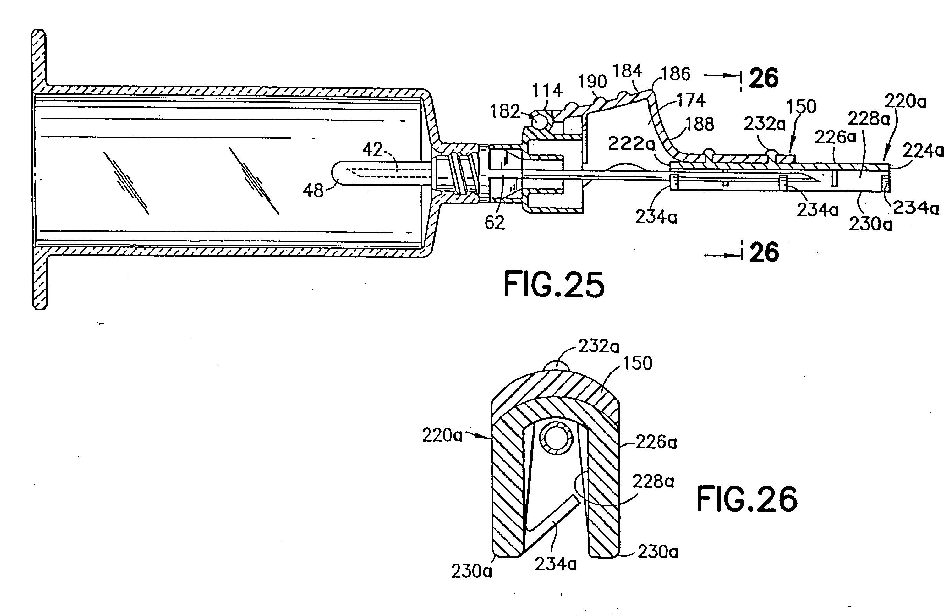

FIGS. 21-26 show an alternate shield **140a**. Shield **140a** comprises a rearward end **144** and a forward end **146a**. Forward end **146a** of shield **140a** defines an elongate support wall **150** with a distal end **152** and top and bottom surfaces **154** and **156** extending proximally from distal end **152**. In the illustrated embodiment, top and bottom surfaces **154** and **156** are slightly arcuate from side-to-side. However, in other embodiments, top surface **154** and/or bottom surface **156** may be substantially planar. Support wall **152** is characterized by axially spaced apertures **158** extending entirely therethrough from top surface **154** to bottom surface **156**.

Rearward end **144** of shield **140a** is substantially identical to rearward end **144** of shield **140**. Thus, a repetitive description is not provided and corresponding elements merely are identified by the same reference numerals.

Shield **140a** further includes a cannula channel **220a**. Cannula channel **220a** includes opposed longitudinal ends **222a** and **224a**, an elongate convex outer surface **226a** extending between ends **222a** and **224a** and an inner concave surface **228a** extending between ends **222a** and **224a**. An entry to concave inner surface **228a** is defined by opposed parallel edges **230a** that extend substantially between ends **222a** and **224a** of channel **220a**. Mounting projections **232a** project from convex outer surface **226a** at a location substantially equally spaced from edges **230a**. Thus, mounting projections **232a** are substantially opposite from the entry into concave inner surface **228a** of channel **220a**. Mounting projections **232a** are spaced from one another by a distance substantially equal to the spacing between mounting apertures **158** in support wall **150**. Additionally, mounting projections **232a** are dimensioned to be snapped or force fit into apertures **158**. Mounting projections **232a** are not symmetrically disposed along the length of cannula channel **220a**. Rather, mounting projections **232a** are closer to end **222a** than to end **224a**. Channel **220a** is defined further by cannula finger locks **234a** that project obliquely inwardly from one of side edges **230a**. Cannula finger locks **234a** are resiliently deflectable in response to forces exerted by needle **40** as explained further below.

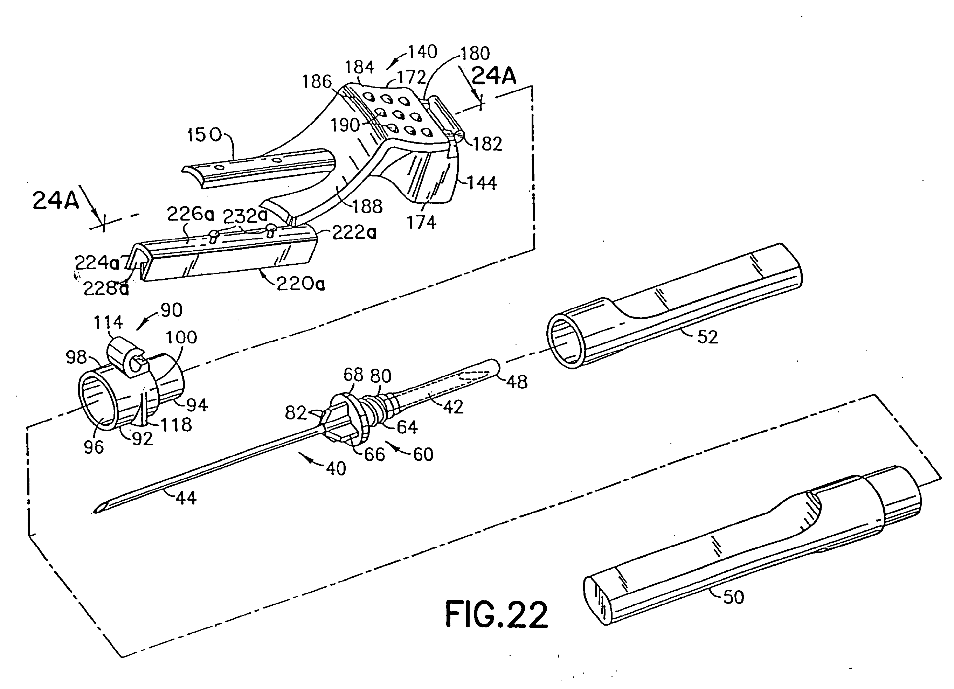

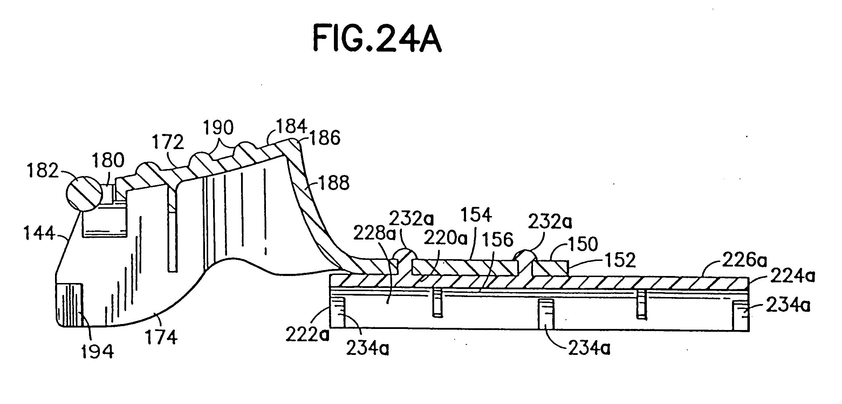

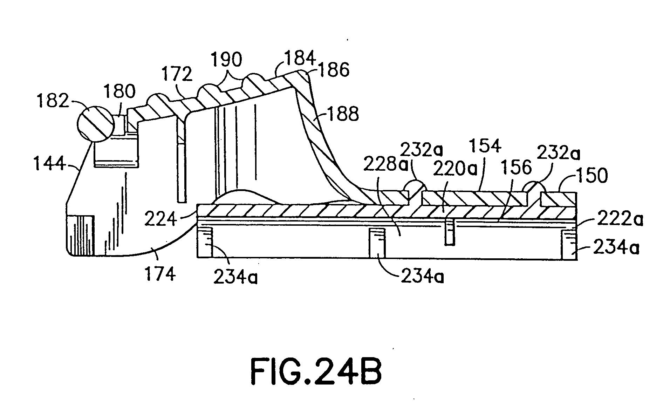

As shown in FIGS. 22, 23A and 24A, channel **220a** can be oriented such that end **224a** of channel **220a** projects distally or forwardly beyond support wall **150**. This orientation is appropriate in situations where it is known that a relatively long needle cannula will be employed with the needle assembly. Alternatively, however, channel **220a** can be oriented as shown in FIG. 24B so that end **222a** of channel **220a** is aligned substantially with the distal end **152** of support wall **152** while end **224a** of channel **222a** projects rearwardly or proximally into rearward end **144** of shield **140a**.

Shield **140a** is used substantially in the same manner as shield **140** described above and illustrated in FIGS. 7-15. More particularly, needle **40** contacts cannula finger locks **234a**. Accordingly, cannula finger locks **234a** deflect toward support wall **150** and away from edges **230a**. Sufficient rotation of shield **140** will cause needle **40** to pass cannula finger locks **234a**. As a result, cannula finger locks **234a** will return resiliently to an undeflected condition. Thus, needle **40** will be trapped above cannula finger locks **234a**.

Needle **40** is contained within shield **140a** as the shield is pivoted into the closed position. More particularly, proximal surfaces **197** of rounded ears **194** move over ramp surfaces **122** of chevron-shaped projection **118** and cause sidewalls **174** to deflect away from one another. The angularly aligned proximal faces **197** of rounded ears **194** ensure easy movement of shield **140a**. Additionally, the resiliency of sidewalls **174** and the angular alignment of distal surface **195** of ears **194** causes a cooperation with rounded proximal ends **124** of chevron-shaped projection **118** to accelerate shield **140a**. This accelerated movement of shield **140a** helps to generate a clear audible and tactile indication of complete shielding.

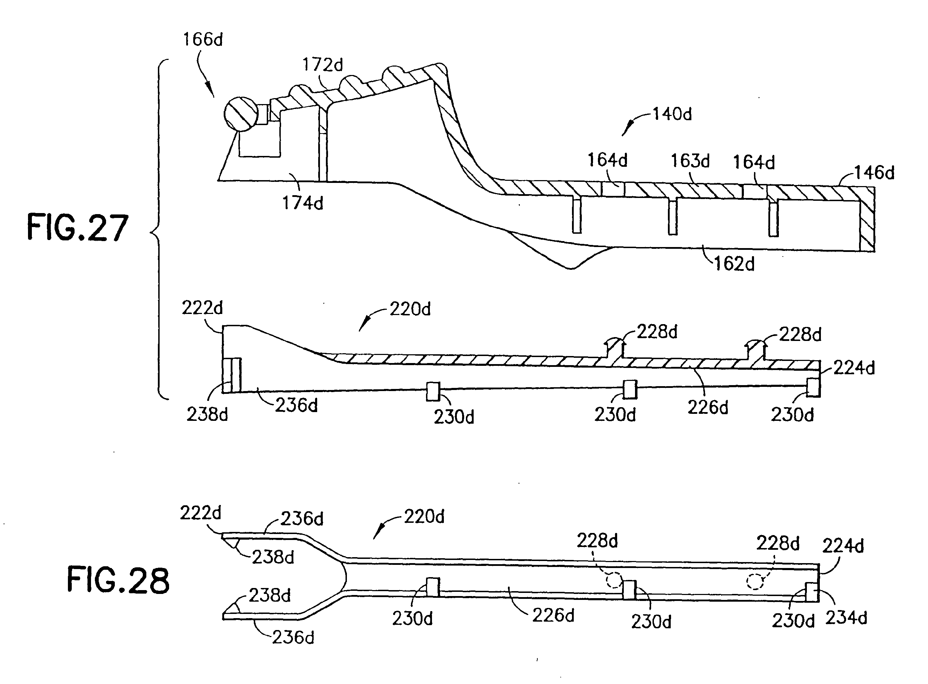

FIGS. 27 and 28 show a further alternate to the needle assemblies described and illustrated above. In particular, the embodiment of FIGS. 27 and 28 includes a shield **140d** that is similar to the shield **140a** described and illustrated above. Shield **140d** includes a collar engaging area 166d with a top finger guide area **172d** and sidewalls **174d** that extend downwardly from top finger guide area **172d**. Sidewalls **174d** differ from the embodiments described and illustrated above in that they extend a shorter distance from top finger guide area **172d** and have no means for engaging the chevron-shaped projection on the collar.

Shield **140d** includes a forward end **146d** with a top wall **163d** and sidewalls **162d** extending down from top wall **163d**. Top wall **163d** is characterized by spaced apart mounting apertures **164d**.

Shield **140d** further includes a channel-shaped clip **220d** having a proximal end **222d**, a distal end **224d** and a top wall **226d** extending from distal end **224d** toward proximal end **222d**. The top wall includes a pair of spaced apart mounting projections **228d** that are dimensioned to be mounted in mounting apertures **158d** on forward end **146d** of shield **140d**. Cannula finger locks **230d** extend from the channel-shaped clip in a direction away from mounting projections **228d**. Each cannula finger lock **230d** includes a support leg and a cannula engaging leg **234d**. Cannula finger locks **230d** are dimensioned and configured to trap the needle cannula when shield **140d** is rotated into the closed position.

Proximal end **222d** of clip **220d** includes a pair of collar engaging legs **236d** extending generally proximally from top wall **226d**. Cannula engaging legs **236d** are configured to pass around opposite respective sides of the collar. Ends of collar engaging legs **236d** furthest from top wall **226d** are formed with rounded ears **238d**. Ears **238d** are configured to snap into engagement with the chevron-shaped projection, as described above.

Shield **140d** is used substantially exactly as shield **140** described above. In particular, shield **140d** can be rotated from the open position to the closed position. As shield **140d** approaches the closed position, cannula engaging legs **234d** of cannula locking fingers locks **230d** engage the needle cannula. Simultaneously, rounded ears **238d** engage the chevron-shaped projection. The rounded configuration of ears **238d** helps to propel shield **140d** toward the closed position. In the closed position, cannula finger locks **230d** will snap past the needle cannula for permanently trapping the needle cannula within shield **140d**.

The shield and collar of the safety shield assembly of the present invention are comprised of moldable parts which can be mass produced from a variety of materials including, for example, polyethylene, polyvinyl chloride, polystyrene or polyethylene and the like. Materials will be selected which will provide the proper covering and support for the structure of the invention in its use, but which will provide also a degree of resiliency for the purpose of providing the cooperative movement relative to the shield and the collar of the assembly.

## Claims

1. A safety needle assembly comprising a needle hub with proximal and distal ends and a passage extending between said ends, a needle cannula mounted to said passage of said needle hub and having a pointed distal end projecting beyond said distal end of said hub, a shield having proximal and distal ends, said proximal end of said shield being hingedly mounted to said hub for rotation from a first position where said shield is spaced from said needle cannula to a second position where said shield substantially shields said needle cannula, said shield comprising at least one support wall, a locking structure mounted to said support wall, said locking structure having a top wall and at least one resiliently deflectable cannula finger lock, said cannula finger lock engaging said needle cannula when said shield is rotated to said second position.

2. The safety needle assembly of claim 1, wherein said top wall of said locking structure comprises mounting members for mounting said locking structure to said support wall, said locking structure having opposite first and second longitudinal ends, said mounting members being closer to said first end of said locking structure than to said second end, whereby said locking structure can be disposed in either a first rotational orientation or in a second rotational orientation relative to said support wall for altering positions of said locking structure relative to said needle cannula.

3. The safety needle assembly of claim 2, wherein said support wall has a distal end remote from said proximal end of said shield, said locking structure being dimensioned such that in said second rotational orientation of said locking structure relative to said support wall, said second end of said locking structure projects distally beyond said distal end of said support wall.

4. The safety needle assembly of claim 3, wherein said locking structure is dimensioned such that in said first rotational orientation, said first end of said locking structure substantially aligns with said distal end of said support wall.

5. The needle assembly of any of claims 2-4, wherein said locking structure is dimensioned such that in said first rotational orientation, said second end of said locking structure is substantially adjacent said proximal end of said shield.

6. The safety needle assembly of any of claims 2-5, wherein said at least one cannula finger lock comprises a plurality of cannula finger locks, a first of said cannula finger locks being substantially adjacent said first end of said locking structure and a second of said cannula finger locks being substantially adjacent said second end of said locking structure.

7. The safety needle assembly of any of claims 1-6, wherein said support wall has a concave surface configured for closely receiving said locking structure.

8. The safety needle assembly of any of claims 1-7, wherein the locking structure is a locking channel having first and second sidewalls extending from said top wall.

9. The safety needle assembly of any of claims 1-8, wherein said support wall comprises a plurality of apertures extending therethrough, said locking structure having a plurality of mounting projections formed thereon, said mounting projections being dimensioned and disposed for locked engagement with said apertures in said support wall.

10. The safety needle assembly of any of claims 1-9, wherein portions of said shield adjacent said proximal end are configured for partly surrounding said hub, said support wall extending substantially rigidly from said portions of said shield dimensioned for partly surrounding said hub.

11. The safety needle assembly of any of claims 1-7, wherein said shield comprises first and second sidewalls extending unitarily from said support wall, said locking structure defining a clip mounted to said support wall and between said sidewalls of said shield.

12. The safety needle assembly of claim 11, further comprising at least one support leg extending from said top wall of said clip, said cannula finger lock projecting from an end of said support leg remote from said top wall of said clip.

13. The safety needle assembly of any of claims 1-12, further comprising a chevron-shaped projection formed externally on said hub, and preferably at a location on said hub substantially opposite said hinged connection of said shield to said hub, said chevron-shaped projection including a central point facing distally on said hub and a pair of rounded ends facing proximally on said hub, rounded ears formed on said shield for engaging said rounded proximal ends of said chevron-shaped projection when said shield is in said second position, whereby said rounded ears and said rounded proximal ends of said chevron-shaped projection provide audible and tactile indication of said shield reaching said second position.

14. The safety needle assembly of claim 13, wherein said rounded ears are formed on inwardly facing surfaces of said sidewalls of said shield, and wherein said rounded ears are dimensioned to require deflection of said sidewalls away from one another as said shield is rotated into said second position and as said rounded ears move over said rounded proximal ends of said chevron-shaped projection.

15. The safety needle assembly of any of claims 13-16, wherein each said rounded ear comprises a proximal face aligned to said respective sidewall at an acute angle, a distal face aligned to said respective side wall at an acute angle of preferably about 60° and a curved surface extending between said proximal and distal faces.
